(19) Europäisches Patentamt
European Patent Office
Office européen des brevets

(11) Veröffentlichungsnummer: **0 206 167**
**B1**

# (12) EUROPÄISCHE PATENTSCHRIFT

(45) Veröffentlichungstag der Patentschrift: **04.01.89**

(51) Int. Cl.⁴: **C 07 C 147/06**, C 07 C 147/14, C 07 C 149/40, A 01 N 53/00

(21) Anmeldenummer: **86108096.8**

(22) Anmeldetag: **13.06.86**

(54) **Tetramethylcyclopropancarbonsäureester.**

(30) Priorität: **25.06.85 DE 3522623**

(43) Veröffentlichungstag der Anmeldung: **30.12.86 Patentblatt 86/52**

(45) Bekanntmachung des Hinweises auf die Patenterteilung: **04.01.89 Patentblatt 89/1**

(84) Benannte Vertragsstaaten: **AT BE CH DE FR GB IT LI NL**

(56) Entgegenhaltungen:
**EP-A-0 031 199**
**EP-A-0 060 617**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Naumann, Klaus, Dr., Richard- Wagner-Strasse 9, D-5090 Leverkusen (DE)**
Erfinder: **Braden, Rudolf, Dr., Nothauser Feld 1, D-5068 Odenthal (DE)**
Erfinder: **Behrenz, Wolfgang, Dr., Untergruendemich 14, D-5063 Overath (DE)**
Erfinder: **Becker, Benedikt, Dr., Metzkausener Strasse 14, D-4020 Mettmann (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

EP 0 206 167 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft einen neuen Tetramethylcyclopropancarbonsäureester, Verfahren zu seiner Herstellung, seine Verwendung als Pflanzenschutzmittel, insbesondere als Insektizid und Akarizid.

Es ist bereits bekannt geworden, daß ähnlich strukturierte Cyclopropancarbonsäureester (z. B. aus EP-A-0 060 617) als Insektizide einsetzbar sind. Diese zeigen jedoch eine erheblich schwacher ausgeprägte Wirksamkeit als die erfindungsgemäße Verbindung.

Es wurde der neue Vinylcyclopropancarbonsäureester der Formel I

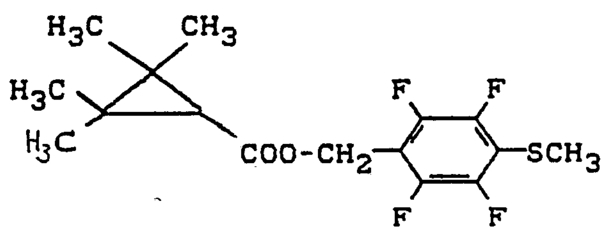

(I)

gefunden.

Man erhält den neuen Tetramethylcyclopropancarbonsäureester der Formel I,

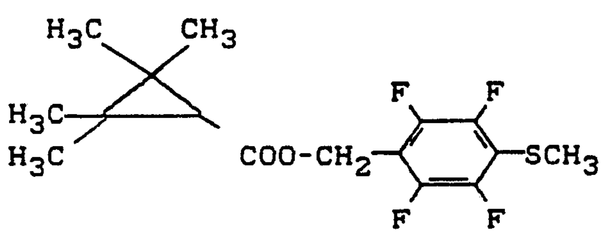

(I)

a) wenn man Tetramethylcyclopropancarbonsäure oder deren reaktionsfähiges Derivat der Formel II

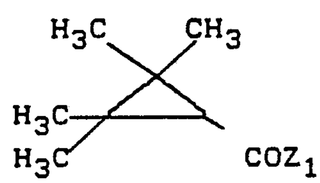

(II)

in welcher $Z_1$ Halogen, vorzugsweise Chlor oder OH, bedeutet, mit dem Alkohol oder dessen reaktionsfähigem Derivat der Formel III

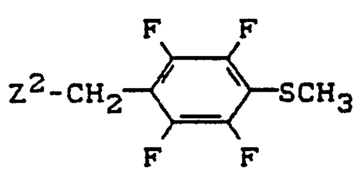

(III)

in welcher R die oben angegebene Bedeutung hat und $Z^2$ für OH, Cl oder Br steht, gegebenenfalls in Gegenwart von Lösungsmitteln, Säureakzeptoren und/oder Phasentransferkatalysatoren umsetzt;

oder

b) wenn man die Verbindung 2,2,3,3-Tetramethylcyclopropancarbonsäurepentafluorbenzylester mit Alkalimetallmethylmercaptid umsetzt.

Vorzugsweise erfolgt die Umsetzung der Verbindungen (II) mit solchen der Formel (III) ohne Anwesenheit von Lösungsmitteln. Die Alkohole bzw. reaktionsfähigen Alkoholderivate der Formel III

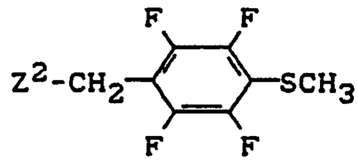

(III)

wobei R und $Z^2$ die oben angegebenen Bedeutungen besitzen, werden hergestellt, indem man Pentaf luorbenzylalkohol oder seine Derivate der Formel (IV)

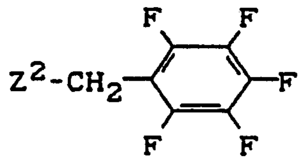

(IV)

wobei $Z^2$ für OH, Cl oder Br steht, gegebenenfalls in Anwesenheit von Säureakzeptoren mit

$CH_3SH$ (V)

umsetzt.

Überraschenderweise zeigt der erfindungsgemäße Cyclopropancarbonsäureester der Formel (I) eine erheblich höhere insektizide Wirkung als die aus dem Stand der Technik bekannten Verbindungen gemäß EP-A-0 060 617.

Verwendet man beispielsweise Tetramethylcyclopropancarbonsäurechlorid und 2,3,5,6-Tetrafluor-4-methylmercaptobenzylalkohol als Ausgangskomponenten, so kann der Reaktionsverlauf durch das folgende Formelschema wiedergegeben werden:

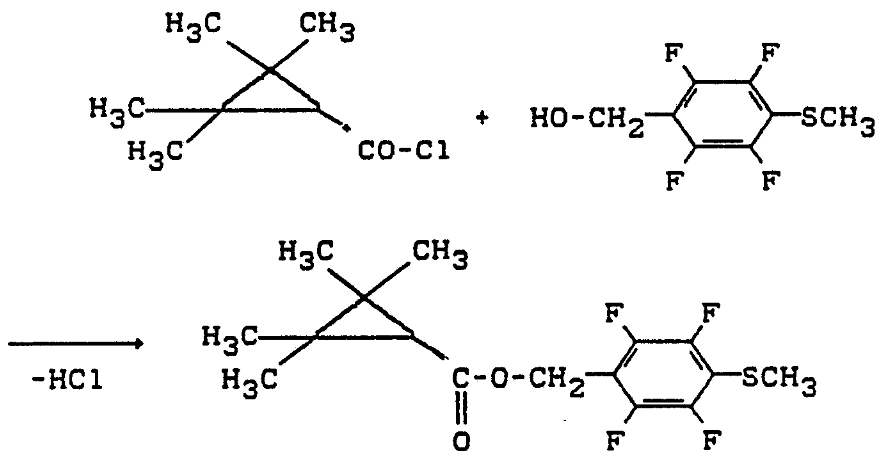


Als Ausgangsprodukte werden Benzylalkohole bzw. deren reaktionsfähigen Derivate der Formel (III) eingesetzt. Als reaktionsfähige Derivate werden bevorzugt die Chloride verwendet.

Als Beispiel für die als Ausgangsprodukte zu verwendenden Verbindungen der Formel III sei genannt:

4-Methylmercapto-2,3,5,6-tetrafluorbenzylalkohol

Die Umsetzung der Säuren bzw. reaktionsfähigen Derivate der Säuren der Formel (II) mit den Alkoholen oder den reaktionsfähigen Derivaten der Alkohole (III) geschieht vorzugsweise in Abwesenheit von Lösungsmitteln. Insbesondere werden so die Säurechloride (Formel II, $Z_1$ = Cl) umgesetzt, wobei man dann bis zum Ende der Chlorwasserstoffentwicklung erwärmt. Natürlich können auf diese Art auch andere Säurehalogenide wie z. B. Säurebromide umgesetzt werden.

Die Aufarbeitung der Reaktionsprodukte erfolgt im allgemeinen durch Destillation.

Zur Herstellung der erfindungsgemäßen Verbindung der Formel I gemäß 1. (oben) aus Carbonsäuren bzw. Carbonsäurehalogeniden der Formel II und Alkoholen bzw. Chloriden oder Bromiden der Formel III können aber auch als Säureakzeptoren, z. B. alle üblichen Säurebindemittel, Verwendung finden.

Besonders bewährt haben sich Alkalihydroxide, -carbonate und -alkoholate, wie Kaliumhydroxid, Natriumhydroxid, Natriummethylat, Kaliumcarbonat, Natriumethylat, ferner aliphatische, aromatische oder heterocyclische Amine, beispielsweise Triethylamin, Trimethylamin, Dimethylanilin, Dimethylbenzylamin und Pyridin.

Die Reaktionstemperatur der Umsetzung von Verbindungen (II) mit Verbindungen (III) kann innerhalb eines

größeren Bereiches variiert werden Im allgemeinen arbeitet man bei der Umsetzung der Säurehalogenide mit Alkoholen zwischen 0 und 100°C, vorzugsweise bei 15 bis 40°C und bei der Umsetzung der Carbonsäure mit den Halogeniden zwischen 50 und 150°C, vorzugsweise bei 80 bis 120°C. Im letzteren Fall wird bevorzugt in Gegenwart eines Katalysators gearbeitet.

Als Katalysatoren kommen alle sogenannten Phasentransferkatalysatoren in Betracht, wie beispielsweise Kronenether oder quartäre Ammonium- oder Phosphoniumsalze. Bevorzugt sind quartäre Ammoniumsalze, wie beispielsweise Tetrabutylammoniumchlorid, Tetrabutylammoniumbromid, Benzltriethylammoniumchlorid oder Methyltrioctylammoniumchlorid.

Die Umsetzung läßt man im allgemeinen bei Normaldruck ablaufen. Das Verfahren zur Herstellung der erfindungsgemäßen Verbindungen wird bevorzugt ohne Mitverwendung von Lösungsmitteln durchgeführt Selbstverständlich kann die Reaktion auch in Gegenwart geeigneter Lösungs- und Verdünnungsmittel durchgeführt werden. Als solche kommen praktisch alle inerten organischen Solventien infrage. Hierzu gehören insbesondere aliphatische und aromatische, gegebenenfalls chlorierte Kohlenwasserstoffe wie Benzol, Toluol, Xylol, Benzin, Methylenchlorid, Chloroform, Dichlorethan, Chlorbenzol, o-Dichlorbenzol oder Ether, z. B. Diethyl-, Diisopropyl- oder Dibutylether, außerdem Nitrile, wie Aceto- und Propionitril.

Eine weitere bevorzugte Herstellungsmethode ist die Umsetzung der Alkalisalze der Säuren mit entsprechenden Benzylhalogeniden der Formel III ($Z^2$ = Cl, Br) in Gegenwart z. B. von katalytischen Mengen Pentamethylethylentriaminen o.ä. Aminen und beispielsweise in Acetonitril wie z. B. in Synthesis 1975, 805, beschrieben.

Zur Durchführung des Verfahrens setzt man die Ausgangskomponenten vorzugsweise in äquimolaren Mengen ein. Die Reaktionskomponenten werden gegebenenfalls in einem der angegebenen Lösungsmittel zusammengegeben und meist bei erhöhter Temperatur nach Zugabe des Säureakzeptors und gegebenenfalls des Katalysators zur Vervollständigung der Reaktion eine oder mehrere Stunden gerührt. Anschließend gießt man das Reaktionsgemisch in Wasser, trennt die organische Phase ab und wäscht diese mit Wasser neutral. Nach dem Trocknen wird das Lösungsmittel im Vakuum abdestilliert.

Eine weitere Methode zur Herstellung von 2,2,3,3-Tetramethylcyclopropancarbonsäuremethylmercaptobenzylester besteht darin, daß man in einem Lösungsmittel vorzugsweise unter Inertgas (insbesondere Stickstoff) 2,2,3,3-Tetramethylcyclopropancarbonsäurepentafluorbenzylester mit Alkalimetallmethylmercatid, vorzugsweise mit Natriummethylmercaptid, umsetzt und die organische Phase destillativ entfernt.

Der Wirkstoff eignet sich bei guter Pflanzenverträglichkeit und günstiger Warmblüteroxizität zur Bekämpfung von tierischen Schädlingen, insbesondere von Insekten und Spinnentieren, die in der Landwirtschaft, in Forsten, im Vorrats- und Matrialschutz sowie auf dem Hyginesektor vorkommen. Er ist gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis,

Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp..

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus,

Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp. , Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp..

Aus der Ordnung der Arachnida z. B. Scorpio maurus, Latrodectus mactans.

Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Der Wirkstoff kann in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z. B. durch Vermischen des Wirkstoffs mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff

und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstof fe für Granulate kommen in Frage:

z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-Ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung des erfindungsgemäßen Wirkstoffs erfolgt in Form seiner handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die Anwendung des erfindungsgemaßen Wirkstoffs geschieht im Veterinärsektor in bekannter Weise, wie

durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

**Beispiel A**

$LD_{100}$-Test
Testtiere: Ceucophea maderae
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man 5 Testtiere in die Petrischale und bedeckt sie mit einem Glasdeckel.

Der Zustand der Testtiere wird 3 Tage nach Ansetzen der Versuche kontrolliert. Bestimmt wird die Abtötung in %.

Bei diesem Test zeigen z. B. die folgenden Verbindungen der Herstellungsbeispiele überlegene Wirksamkeit gegenüber dem Stand der Technik: 1.

**Beispiel B**

$LT_{100}$-Test für Dipteren
Testtiere: 1. Musca domestica, 2. Aedes aegypti
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 25 Testtiere in die Petrischale und bedeckt sie mit einem Glasdeckel.

Der Zustand der Testtiere wird laufend kontrolliert. Es wird diejenige Zeit ermittelt, welche für einen 100-%-igen knock down-Effekt notwendig ist.

Bei diesem Test zeigen z. B. die folgenden Verbindungen der Herstellungsbeispiele eine überlegene Wirksamkeit gegenüber dem Stand der Technik: 1.

**Beispiel C**

Nephotettix-Test
Lösungsmittel: 7 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigen z. B. die folgenden Verbindungen der Herstellungsbeispiele überlegene Wirksamkeit gegenüber dem Stand der Technik: 1.

**Beispiel D**

Tetranychus-Test (resistent)
Lösungsmittel: 7 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 1.

**Beispiel E**

Grenzkonzentrations-Test / Bodeninsekten
Testinsekt: Phorbia antiqua-Maden (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in %. bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber dem Stand der Technik: 1.

**Beispiel F**

Grenzkonzentrations-Test / Bodeninsekten
Testinsekt: Diabrotica balteata - Larven im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (= mg/l) angegeben wird. Man füllt den Boden in 0,5 l Töpfe und läßt diese bei 20° C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z. B. die folgenden Verbindungen der Herstellungsbeispiele überlegene Wirksamkeit gegenüber den Stand der Technik: 1.

**Beispiel 1**

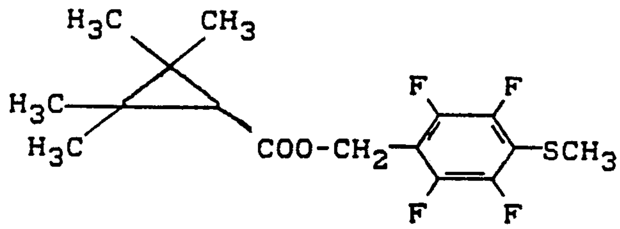

0,1 Mol (16 g) Tetramethylcyclopropancarbonsäurechlorid und 0,1 Mol (22,6 g) 2,3,5,6-Tetrafluor-4-methylmercaptobenzylalkohol wurden zusammen ohne Lösungsmittel auf 50 bis 70°C bis zum Ende der Chlorwasserstoffentwicklung erwärmt. Das Produkt wurde anschließend im Vakuum destilliert. Man erhielt 39 g Verbindung der obenstehenden Formel (Fp.: Kp 0,1: 120°C)

IR-Spektrum: 2950 1730 1640 1470 1420 1330 1270 1190 1140 1120 1070 1050 910 870

Erläuterung: Der Ausdruck "Kp 0,1" bedeutet hier und im folgenden Siedepunkt bei 0,1 mm Quecksilbersäule.

**Beispiel 2**

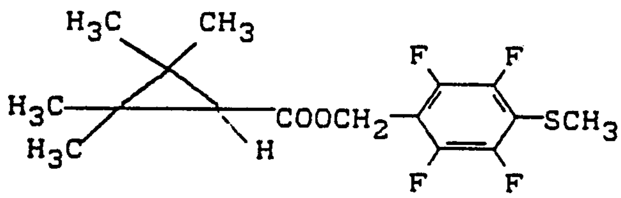

Zu 0,1 Mol 2,2,3,3-Tetramethylcyclopropancarbonsäurepentafluorbenzylester in 150 ml Methylenchlorid werden bei 20°C 0,1 Mol Natriummethylmercaptid unter Stickstoff zugetropft. Nach Eintreten der Neutralreaktion wird die organische Phase am Kugelrohr im Vakuum destilliert (230°C Ofentemperatur, 0,05 mm). Man erhält laut NMR-Spektrum reine Titelverbindung 2,2,3,3-Tetramethylcyclo-propancarbonsäure-tetrafluor-4-methylmercaptobenzylester.

**Ausgangsmaterial**

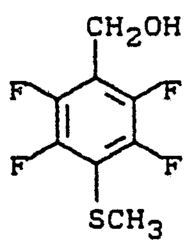

In einer 250 ml-Dreihalskolbenrührapparatur mit Thermometer, Kühler und Kühlbad werden 100 ml Isopropanol bei 0°C vorgelegt, dann werden 5 g Methylmercaptan eingeleitet und 4 g gepulvertes Natriumhdroxid zugegeben. Danach werden in 15 Minuten über einen beheizbaren Tropftrichter bei 0°C 20 g Pentafluorbenzylalkohol zugetropft. Anschließend wird langsam auf Rückflußtemperatur (83 - 84°C) erwärmt und eine Stunde bei dieser Temperatur gerührt.

Der Ansatz wird abgekühlt und auf Eiswasser gegeben. Die entstehenden schmierigen Kristalle werden in Methylenchlorid aufgenommen.

Anschließend werden die beiden Phasen getrennt, die organische phase über Natriumsulfat getrocknet und an einer Kolonne destilliert. Man erhält 16,6 g 2,3,5-Tetrafluor-4-methylmercaptobenzylalkohol (Kp. 16 mbar: 145 - 146° C).

**Patentansprüche**

1. Tetramethylcyclopropancarbonsäureester der Formel (I)

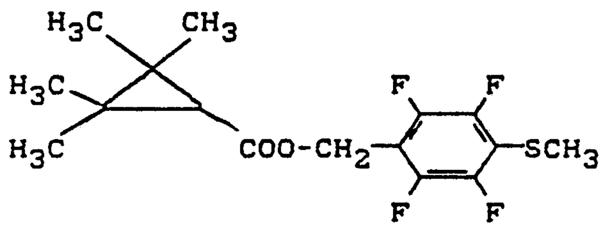

(I)

2. Verfahren zur Herstellung des Tetramethylcyclopropancarbonsäureester der Formel (I)

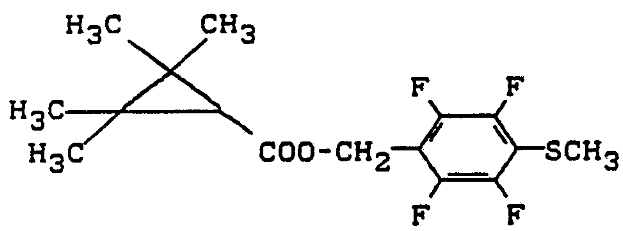

(I)

dadurch gekennzeichnet, daß man

a) die Säure oder deren reaktionsfähiges Derivat der Formel (II)

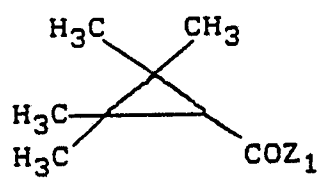

(II)

in welcher

$Z_1$ Halogen oder OH bedeutet,

mit dem Alkohol oder dessen reaktionsfähigem Derivat der Formel (III)

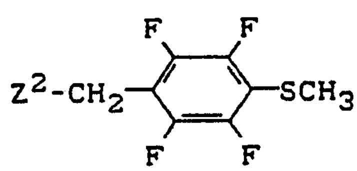

(III)

in welcher

$Z^2$ für OH, Cl oder Br steht,

gegebenenfalls in Gegenwart von Lösungsmitteln, Säureakzeptoren und/oder Phasentransferkatalysatoren umsetzt;

oder

b) daß man die Verbindung 2,2,3,3-Tetramethylcyclopropancarbonsäurepentafluorbenzylester mit Alkalimetallmethylmercaptid umsetzt.

3. Schädlingsbekämpfungsmittel, gekennzeichnet durch einen Gehalt an dem Tetramethylcyclopropancarbonsäureester der Formel (I).

4. Verfahren zur Bekämpfung von tierischen Schädlingen, dadurch gekennzeichnet, daß man Tetramethylcyclopropancarbonsäureester der Formel (I) auf tierische Schädlinge und/oder ihren Lebensraum einwirken läßt.

5. Verwendung des Tetramethylcyclopropancarbonsäureesters der Formel (I) zur Bekämpfung von tierischen Schädlingen.

6. Verfahren zur Herstellung von Schädlingsbekämpfungsmitteln, dadurch gekennzeichnet, daß man den Tetramethylcyclopropancarbonsäureester der Formel (I) mit Streckmitteln und/oder oberflächenaktiven Mitteln vermischt.

## Claims

1. Tetramethylcyclopropanecarboxylate of the formula (I)

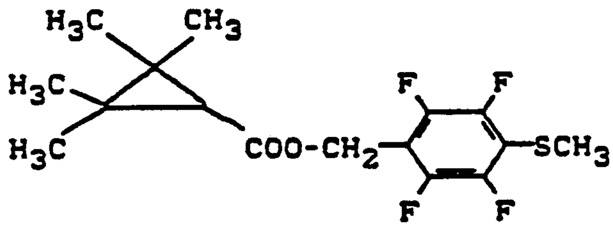

(I)

2. Process for the preparation of tetramethylcyclopropanecarboxylates of the formula (I)

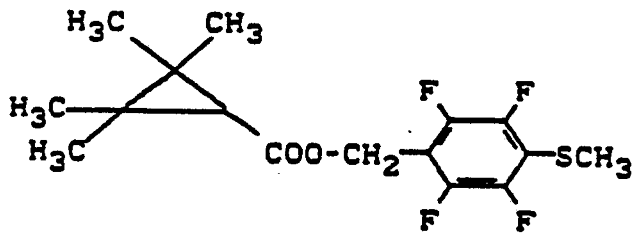

(I)

characterized in that

(a) the acid or its reactive derivative of the formula II

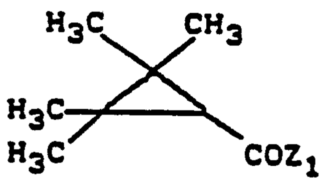

(II)

in which

$Z_1$ denotes halogen or OH,

is reacted with an alcohol or its reactive derivative of the formula (III)

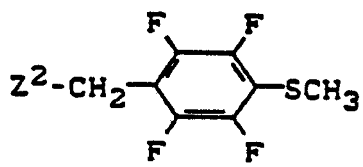

(III)

in which

$Z^2$ represents OH, Cl or Br,

if appropriate in the presence of solvents, acid acceptors and/or phase-transfer catalysts;

or

(b) the compound pentafluorobenzyl 2,2,3,3-tetramethyl-cyclopropanecarboxylate is reacted with alkali metal methanethiolate.

3. Pest-combatting agents, characterized in that they contain the tetramethylcyclopropanecarboxylate of the formula (I).

4. Method for combatting animal pests, characterized in that the tetramethylcyclopropanecarboxylates of the formula (I) are allowed to act on animal pests and/or their habitat.

5. Use of the tetramethylcyclopropanecarboxylate of the formula (I) for combatting animal pests.

6. Process for the preparation of pest-combatting agents, characterized in that the tetramethylcyclopropanecarboxylate of the formula (I) is mixed with extenders and/or surface-active agents.

**Revendications**

1.Ester d'acide tétraméthylcyclopropane-carboxylique de formule (I)

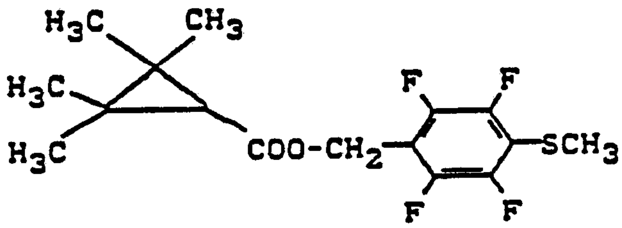

(I)

2. Procédé de fabrication d'ester d'acide tétramethylcyclopropane-carboxylique de formule (I)

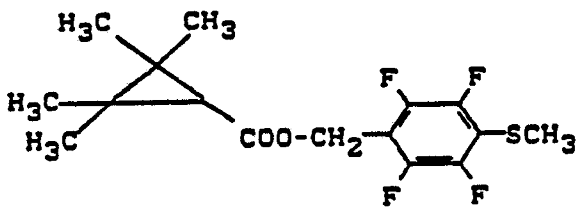

(I)

caractérisé en ce que l'on fait réagir
a) l'acide ou son dérivé réactif de formule (II)

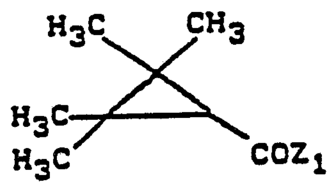

(II)

dans laquelle
$Z_1$ représente un halogène ou un groupe OH,
avec l'alcool ou son dérivé réactif de formule (III)

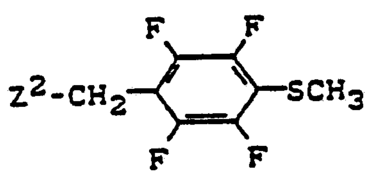

(III)

dans laquelle
$Z^2$ représente un substituant OH, Cl ou Br,
éventuellement en présence de solvants, avec des fixateurs d'acide et/ou des catalyseurs de transfert de phase;
ou
b) le composé ester pentafluorobenzylique d'acide 2,2,3,3-tétraméthylcyclopropane-carboxylique avec un méthylmercaptide de métal alcalin.

3. Produit antiparasite caractérisé en ce qu'il contient l'ester d'acide tétraméthylcyclopropane carboxylique de formule (I).

4. Procédé destiné à lutter contre les animaux parasites caractérisé en ce que l'on fait agir l'ester d'acide tétraméthylcyclopropane-carboxylique de formule (I) sur les animaux parasites et/ou sur leur environnement.

5. Utilisation de l'ester d'acide tétraméthylcyclopropane-carboxylique de formule (I) pour lutter contre les animaux parasites.

6. Procédé de fabrication de produits antiparasites caractérisé en ce que l'on mélange l'ester d'acide tétraméthylcyclopropane-carboxylique de formule (I) avec des diluants et/ou des agents tensio-actifs.